# EUROPEAN PATENT SPECIFICATION

(11) **EP 3 731 819 B1**
(45) Date of publication and mention of the grant of the patent: **26.08.2026**
(21) Application number: 18918421.1
(22) Date of filing: 28.12.2018
(51) Int. Cl.: A61K 9/16, A61K 31/606, A61K 9/50

(54) **ORAL PHARMACEUTICAL COMPOSITIONS OF MESALAZINE**
ORALE PHARMAZEUTISCHE ZUSAMMENSETZUNGEN VON MESALAZIN
COMPOSITIONS PHARMACEUTIQUES ORALES DE MÉSALAZINE

(30) Priority: 29.12.2017 TR 201722852
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); TASKIN, Abdullah, 34460 Istanbul (TR); DEDEOGLU, Yavuz, 34460 Istanbul (TR)
(74) Representative: Genç Ilhan, Oznur
(86) International application number: PCT/TR2018/050930
(87) International publication number: WO 2019/240700

(56) References cited:
- EP-A1- 1 172 100
- WO-A1-2011/015964
- WO-A1-2017/134418
- WO-A2-2004/093884
- US-A1- 2015 044 289
- US-A1- 2015 306 170
- US-A1- 2016 095 928

## Description

### Field of Invention

The present invention relates to oral pharmaceutical compositions comprising mesalazine or a pharmaceutically acceptable salt thereof wherein the composition is free of polyvinylpyrrolidone, and wherein the amount of mesalazine is between 20-50% by weight of the composition.

### Background of Invention

Aminosalicylates are a group of medicines that can help to control the symptoms of some inflammatory bowel (gut) diseases. They are mainly used to help keep people with ulcerative colitis in remission and to treat flare-ups of ulcerative colitis. There is also some evidence that if you have ulcerative colitis then taking an aminosalicylate on a long-term basis can significantly reduce your risk of developing bowel cancer.

They include balsalazide, mesalazine, olsalazine and sulfasalazine, and come in a variety of different forms as tablets or liquid to be taken by mouth, liquid or foam enemas, suppositories to be inserted into the rectum.

All of the aminosalicylate medicines contain the active ingredient called 5-aminosalicylic acid (5-ASA). Mesalazine is an agent derived from sulfasalazine. Its chemical name is 5-amino-2-hydroxybenzoic acid and its chemical structure is shown in the Formula 1.

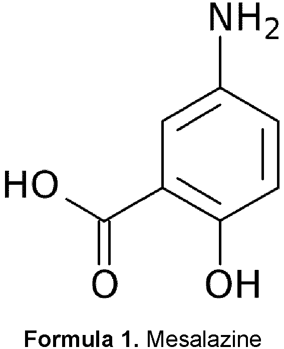

Mesalazine is a molecule with antiinflammatory activity, widely used for the treatment of chronic inflammatory diseases of the intestinal tract. Chronic intestinal inflammatory diseases are a group of inflammatory diseases, with acute or subtle onset, which mainly but not exclusively involve the intestine; they have chronic course and fluctuating activities and progressions over time. In this disease group, the most important are ulcerative colitis and Crohn's disease, serious and disabling diseases, which negatively affect the quality of life of the patients in addition to their health.

Mesalazine is normally administered orally or rectally; in particular, most of the oral compositions currently present on the market are formulated in tablets or granules. The oral forms currently present on the market are mainly formulated in such a manner that the active principle goes beyond the stomach, and often also beyond the small intestine, so as to be essentially released in a site-specific manner at the site of the inflammation, where it acts in a topical manner in direct contact with the mucosa. In order to obtain such site-specificity and the relative topical effectiveness, it is known that the oral forms of mesalazine are coated by one or more layers which allow controlling its release.

Mesalazine comes in six different brand names: Asacol^{®}, Ipocol^{®}, Octasa^{®}, Mezavant^{®}, Pentasa^{®}, Salofalk^{®}. They are all different in terms of manufacturing process and mechanism of action. Tablet forms marketed under the name Asacol^{®}, Ipocol^{®}, Mesren^{®} and Salofalk^{®} have special coatings which dissolve to release the active ingredient (5-ASA) at a certain pH within the gut. On the other hand, each Pentasa^{®} tablet is made up of tiny granules and the active ingredient is gradually released over the length of the gut.

One of the problems about mesalazine tablets in the art is their enlarged sizes by more than one enteric coatings. Therefore, granule forms are more preferable by the patients with swallowing difficulties. However, it is a fact that a granulate dosage form has a larger total surface area comparing to a tablet dosage form and the increase in the surface area accelerates the disintegration of the enteric coating and the core. That can make the whole drug delivery process quite risky unless an enduring enteric coating is provided, since one of the most important objectives for mesalazine formulations is to ensure the release of the active agent at the specified site of delivery.

Therefore, the selection of the type, amount and layer number of the enteric coating plays a crucial role for bioavailability and stability of the formulations especially granulate formulations.

It is also difficult to ensure the stability of granulate mesalazine formulations during the shelf-life, since the enlarged surface area can be a disadvantage depending on the increase of the interaction with the atmospheric moisture. Accordingly, it can be said that the enteric coating becomes even more important for the granulate formulations. One of the parameters providing stable granules is the selection of the manufacturing method which is compatible with the components and accordingly which enhances the adherence of the coating on the core.

The prior art discloses mesalazine granulate formulations with one layer of enteric coating comprising polymethacrylates which are known to be used as binders in wet-granulation processes in the common knowledge (Handbook of Pharmaceutical Excipients edited by Raymond C. Rowe, Paul J. Sheskey and Marlan E. Quinn, sixth edition, page 526). Still in the art, the granules are prepared by the wet granulation of mesalazine with a granulation liquid consisting polyvinylpyrrolidone.

At this point, another issue to consider is the compatibility between the content of the granule core, the enteric coating and the granulation method.

According to the common knowledge again, polyvinylpyrrolidone is a binder specifically suitable for use in wet granulation (Handbook of Pharmaceutical Excipients edited by Raymond C. Rowe, Paul J. Sheskey and Marlan E. Quinn, sixth edition, page 581). It is also known that wet granulation is a complicated and time-consuming method since it requires drying step afterward. Therefore, the usage of polymethacrylates and polyvinylpyrrolidone limits and complicates the manufacturing process.

WO 2017/134418 A1 relates to solid pharmaceutical oral dosage formulations and pharmaceutical filaments for the manufacture of said dosage formulations. According to the teaching of WO 2017/134418 A1; pharmaceutical filaments may be manufactured by hot-melt extrusion method. WO 2017/134418 A1 refers to anti-inflammatory drugs, immunosuppressants, steroids, and antineoplastic drugs as a drug to be used. Mesalazine is cited among dozens of alternatives; while the specifically referenced active agent is paracetamol. According to WO 2017/134418 A, the active agent is present in an amount of 1-15% by weight of the composition.

WO 2011/015964 A1 relates to controlled release granular composition of mesalazine wherein said composition comprises a central core containing an inert substrate; an intermediate layer comprising mesalazine and one or more physiologically acceptable excipients; and a gastro resistant coating. On page 4 of WO 2011/015964 A1; it is clearly disclosed that; mesalazine is applied on a central core comprising a suitable substrate. WO 2011/015964 A1 refers to "suitable substrates" by indicating that such substrates are preferably inert substances, more preferably diluents, optionally with disintegration properties. WO 2011/015964 A1 also highlights on page 4 of the description that; the term "inert" refers to a substrate or core without active principle.

Consequently, it is clear that there is no teaching, suggestion or motivation in the prior art about how to develop a stable mesalazine granulate formulation enabling a simple manufacturing process and ensuring the release of the active agent at the specified site of delivery at the same time. Accordingly, there is still a need for stable mesalazine tablet formulations eliminating all aforesaid problems and bringing additional advantages to the relevant prior art.

### Objects and Brief Description of the Invention

The main object of the present invention is to obtain oral pharmaceutical compositions of mesalazine eliminating all aforesaid problems and bringing additional advantages to the relevant prior art.

Another object of the present invention is to develop granulate compositions of mesalazine with at least one enteric coating ensuring the delivery of the active agent in the colon (large bowel).

Another object of the present invention is to obtain granulate compositions of mesalazine with enhanced stability and disintegration profile.

A further object of the present invention is to develop granulate compositions of mesalazine providing an improved level of dissolution rate and solubility at the specified site of delivery.

Another object of the present invention is to develop granulate compositions of mesalazine which is free of polyvinylpyrrolidone.

A further object of the present invention is to develop granulate compositions of mesalazine which is free of acrylic acid, methacrylic acid a.

Another object of the present invention is to improve a process for preparing the said mesalazine granulate compositions, comprising hot-melt extrusion and spray granulation which provides easy and fast manufacturing.

Yet another object of the present invention is to develop granulate compositions of mesalazine which are suitable for being packed in sachet form.

### Detailed Description of the Invention

In accordance with the objects outlined above, detailed features of the present invention are given herein.

The present invention relates toan oral pharmaceutical composition in the form of granulate which is free of polyvinylpyrrolidone, comprising mesalazine or a pharmaceutically acceptable salt thereof in an amount of between 20-50% by weight of the composition; wherein the granulate composition comprises granule cores comprising mesalazine and an enteric coating on them and wherein the enteric coating is comprised of an inner layer containing hypromellose acetate succinate and an outer layer containing hypromellose acetate succinate..

According to the preferred embodiment, the composition is also free of acrylic acid a, methacrylic acid.

According to one embodiment, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, granules, granulate, powder, coated bead system, microsphere, tablet in tablet, inlay tablet, dragee, sachet or orally administrable film.

According to the present invention, the composition is in the form of granulate. According to the most preferred embodiment, the granulate composition is suitable for being packed in a sachet providing a barrier to humidity, atmospheric air and light.

According to the invention, the granulate composition comprises granule cores comprising mesalazine and enteric coating on them.

According to the invention, the enteric coating is comprised of two layers which are the inner layer and the outer layer. According to this embodiment, the inner layer is obtained by hot-melt extrusion granulation and the outer layer is obtained by spray drying granulation.

According to the invention, the inner layer comprises hypromellose acetate succinate (HPMC AS), preferably HPMC AS-HG.

HPMC AS-HG is a partially esterified derivative of hypromellose in which succinoyl and acetyl residues are bound to the cellulose backbone. It has a pH solubility greater than or equal to 6.5 and its mean particle size is ranging between 0,005-1 mm.

According to the invention, the outer layer comprises hypromellose acetate succinate (HPMC AS), preferably HPMC AS-MG.

HPMCAS-MG, which has a pH solubility greater than or equal to 6.0, is also a partially esterified derivative of hypromellose. Its mean particle size is ranging between 0,005-1 mm.

According to the preferred embodiment, the ratio of HPMC AS-HG to HPMC AS-MG is in the range of 20:1 - 0.5:1, preferably 10:1 - 1:1, more preferably 5:1 - 3:1 by weight.

It has been seen that the synergistic effect of hypromellose acetate succinate as the enteric coating and selection of the granulation methods surprisingly enhances the coating adherence on the core granule, bioavailability and the disintegration rate. Moreover, a surprisingly stable enteric coating is provided due to the coordinated presence of the inner layer and the outer layer with the ratios given above. The desired site of delivery of mesalazine, which is the colon in the gastrointestinal tract, is also ensured between these ranges.

According to the invention, the amount of mesalazine is between 20-50% by weight of the composition.

According to the preferred embodiment, the amount of enteric coating is between 10-90%, preferably 15-80%, more preferably 20-70% by weight of the composition.

According to the preferred embodiment, the granulate composition further comprises at least one excipient selected from sweetening agent, suspending agent, buffer agent, flavoring agent, glidant, diluent, binder, coloring agent or mixtures thereof.

According to one embodiment, the pharmaceutical composition comprises at least one sweetening agent which is selected from the group comprising acesulfame potassium, aspartame, saccharin, saccharin calcium, saccharin sodium, dextrose, fructose, maltitol, mannitol, sorbitol, sucralose, sucrose and mixtures thereof.

According to the preferred embodiment, the composition comprises one sweetening agent which is sucralose.

The amount of sweetening agent is between 1-20%, preferably 3-10% by weight of the composition.

According to one embodiment, the pharmaceutical composition comprises at least one suspending agent which is selected from the group comprising alginates, methylcellulose, hydroxyethylcellulose, carboxymethylcellulose, sodium carboxymethylcellulose, microcrystalline cellulose, acacia, tragacanth, xanthan gum, guar gum, agar, bentonite, carbomer, carrageenan, powdered cellulose, gelatin and mixtures thereof.

According to the preferred embodiment, the composition comprises one suspending agent which is xanthan gum.

The amount of suspending agent is between 0.01-5%, preferably 0.05-1%, more preferably 0.1-0.5% by weight of the composition.

According to one embodiment, the pharmaceutical composition comprises at least one buffer agent which is selected from the group comprising glycine, sodium carbonate, alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate, glycine , glutamic acid and mixtures thereof.

According to the preferred embodiment, the composition comprises one buffer agent which is citric acid anhydrous.

The amount of buffer agent is between 0.01-5%, preferably 0.05-1%, more preferably 0.1-0.5% by weight of the composition.

According to one embodiment, the pharmaceutical composition comprises at least one glidant which is selected from the group comprising calcium stearate, colloidal silicon dioxide, magnesium stearate, sodium stearyl fumarate, sodium lauryl sulphate, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin and mixtures thereof.

According to the preferred embodiment, the composition comprises one glidant which is colloidal silicon dioxide.

The amount of glidant is between 0.01-5%, preferably 0.05-3%, more preferably 0.1-1% by weight of the composition.

According to one embodiment, the pharmaceutical composition comprises at least one diluent which is selected from the group comprising lactose monohydrate, lactose, dibasic calcium phosphate, microcrystalline cellulose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate and mixtures thereof.

According to the preferred embodiment, the composition comprises one diluent which is microcrystalline cellulose.

The amount of diluent is between 1-30%, preferably 5-25%, more preferably 10-20% by weight of the composition.

According to one embodiment, the pharmaceutical composition comprises at least one binder which is selected from the group comprising carnauba wax, hydroxypropyl methyl cellulose (HPMC), pullulan, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose, microcrystalline cellulose, polyethylene oxide, polyvinyl alcohol, polycarbophil, polyvinyl acetate and its copolymers, gelatin, starch, pregelatinized starch, xanthan gum, guar gum, alginate, carrageen, collagen, agar, pectin, hyaluronic acid, carbomer, cellulose acetate phthalate, hydroxypropyl starch, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose and mixtures thereof.

According to the preferred embodiment, the composition comprises one binder which is hydroxypropyl methyl cellulose.

The amount of binder is between 1-30%, preferably 3-20%, more preferably 5-10% by weight of the composition.

According to one embodiment, the pharmaceutical composition comprises at least one flavoring agent in the amount of 0.1-10%, preferably 0.5-5% by weight of the composition.

According to one embodiment, the pharmaceutical composition comprises at least one coloring agent in the amount of 0.1-5%, preferably 0.5-3% by weight of the composition. According to the preferred embodiment, titanium dioxide is present in the composition as the coloring agent.

According to one embodiment, the granulate composition comprises;
- 20-50% by weight of mesalazine,
- 10-90% by weight of hypromellose acetate succinate having a pH solubility greater than or equal to 6.5,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent,
- 0.1-5% by weight of titanium dioxide

According to the preferred embodiment, the granulate composition is packed in a sachet.

The granulate composition mentioned above is prepared by following these steps:
- Obtaining enteric-coated granules by the hot-melt extrusion granulation of mesalazine with hypromellose acetate succinate having a pH solubility greater than or equal to 6.5
- Sieving the enteric-coated granules through a 200µm to 600µm mesh sieve
- Mixing the sieved enteric-coated granules with sucralose, xanthan gum, citric acid anhydrous, colloidal silicon dioxide, flavoring agent and titanium dioxide
- Dosing and filling the granulate into sachets

According to another embodiment, the granulate composition comprises;
- 20-50% by weight of mesalazine,
- 5-60% by weight of hypromellose acetate succinate having a pH solubility greater than or equal to 6.5,
- 5-30% by weight of hypromellose acetate succinate having a pH solubility greater than or equal to 6.0,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent,
- 0.1-5% by weight of titanium dioxide

According to the preferred embodiment, the granulate composition is packed in a sachet.

The granulate composition mentioned above is prepared by following these steps:
- Obtaining single-layered enteric-coated granules by the hot-melt extrusion granulation of mesalazine with hypromellose acetate succinate having a pH solubility greater than or equal to 6.5
- Sieving the enteric-coated granules through a 200µm to 600µm mesh sieve
- Forming another layer of enteric coating on the sieved single-layered enteric-coated granules by spray drying granulation with hypromellose acetate succinate having a pH solubility greater than or equal to 6.0
- Mixing the double-layered enteric-coated granules with sucralose, xanthan gum, citric acid anhydrous, colloidal silicon dioxide, flavoring agent and titanium dioxide
- Dosing and filling the granulate into sachets

According to another embodiment, the composition comprises;
- 20-50% by weight of mesalazine,
- 1-30% by weight of microcrystalline cellulose,
- 1-30% by weight of hydroxypropyl methyl cellulose,
- 10-90% by weight of hypromellose acetate succinate having a pH solubility greater than or equal to 6.5,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent

According to the preferred embodiment, the granulate composition is packed in a sachet.

The granulate composition mentioned above is prepared by following these steps:
- Mixing mesalazine and microcrystalline cellulose
- Granulating this mixture by the addition of hydroxypropyl methyl cellulose to obtain granule cores
- Obtaining enteric-coated granules by the spray drying coating of the granule cores with hypromellose acetate succinate having a pH solubility greater than or equal to 6.5
- Sieving the enteric-coated granules through a 200µm to 600µm mesh sieve
- Mixing the sieved enteric-coated granules with sucralose, xanthan gum, citric acid anhydrous, colloidal silicon dioxide and flavoring agent
- Dosing and filling the granulate into sachets

According to all these embodiments, the below given formulation examples 2-4 can be used in the granulate composition subjected to the invention. Example 1 is a not according to the claimed invention.

### Example 1: Granulate composition comprising single-layered enteric-coated granules

| **Granule core ingredients** | **Amount (%)** |
|---|---|
| Mesalazine | 20 - 50 |

| **Enteric coating ingredients** | **Amount (%)** |
|---|---|
| Inner layer (HPMC AS-HG) | 20-70 |

| **Granulate excipients** | **Amount (%)** |
|---|---|
| Sucralose | 5-10 |
| Xanthan gum | 0.1-0.5 |
| Citric acid anhydrous | 0.1-0.5 |
| Colloidal silicon dioxide | 0.1-1 |
| Flavoring agent | 0.5-5 |
| Titanium dioxide | 0.5-3 |
| **TOTAL** | **100** |

The granulate composition mentioned in Example 1 is prepared by following these steps:
- Obtaining enteric-coated granules by the hot-melt extrusion granulation of mesalazine with hypromellose acetate succinate having a pH solubility greater than or equal to 6.5
- Sieving the enteric-coated granules through a 200µm to 600µm mesh sieve
- Mixing the sieved enteric-coated granules with sucralose, xanthan gum, citric acid anhydrous, colloidal silicon dioxide, flavoring agent and titanium dioxide
- Dosing and filling the granulate into sachets

### Example 2: Granulate composition comprising double-layered enteric-coated granules

| **Granule core ingredients** | **Amount (%)** |
|---|---|
| Mesalazine | 20 - 50 |

| **Enteric coating ingredients** | **Amount (%)** |
|---|---|
| Inner layer (HPMC AS-HG) | 15-50 |
| Outer layer (HPMC AS-MG) | 5-20 |

| **Granulate excipients** | **Amount (%)** |
|---|---|
| Sucralose | 5-10 |
| Xanthan gum | 0.1-0.5 |
| Citric acid anhydrous | 0.1-0.5 |
| Colloidal silicon dioxide | 0.1-1 |
| Flavoring agent | 0.5-5 |
| Titanium dioxide | 0.5-3 |
| **TOTAL** | **100** |

### Example 3: Granulate composition comprising double-layered enteric-coated granules

| **Granule core ingredients** | **Amount (%)** |
|---|---|
| Mesalazine | 40.39 |

| **Enteric coating ingredients** | **Amount (%)** |
|---|---|
| Inner layer (HPMC AS-HG) | 40.39 |
| Outer layer (HPMC AS-MG) | 9.69 |

| **Granulate excipients** | **Amount (%)** |
|---|---|
| Sucralose | 6.06 |
| Xanthan gum | 0.24 |
| Citric acid anhydrous | 0.24 |
| Colloidal silicon dioxide | 0.32 |
| Flavoring agent | 1.62 |
| Titanium dioxide | 1.05 |
| **TOTAL** | **100.00** |

The granulate compositions mentioned in Examples 2 and 3 are prepared by following these steps:
- Obtaining single-layered enteric-coated granules by the hot-melt extrusion granulation of mesalazine with hypromellose acetate succinate having a pH solubility greater than or equal to 6.5
- Sieving the enteric-coated granules through a 200µm to 600µm mesh sieve
- Forming another layer of enteric coating on the sieved single-layered enteric-coated granules by spray drying granulation with hypromellose acetate succinate having a pH solubility greater than or equal to 6.0
- Mixing the double-layered enteric-coated granules with sucralose, xanthan gum, citric acid anhydrous, colloidal silicon dioxide, flavoring agent and titanium dioxide
- Dosing and filling the granulate into sachets

### Example 4: Granulate composition comprising single-layered enteric-coated granules

| **Granule core ingredients** | **Amount (%)** |
|---|---|
| Mesalazine | 20 - 50 |
| Microcrystalline cellulose | 10-20 |
| Hydroxypropyl methyl cellulose | 5-10 |

| **Enteric coating ingredients** | **Amount (%)** |
|---|---|
| Inner layer (HPMC AS-HG) | 20-70 |

| **Granulate excipients** | **Amount (%)** |
|---|---|
| Sucralose | 5-10 |
| Xanthan gum | 0.1-0.5 |
| Citric acid anhydrous | 0.1-0.5 |
| Colloidal silicon dioxide | 0.1-1 |
| Flavoring agent | 0.5-5 |
| **TOTAL** | **100** |

The granulate composition mentioned in Example 4 is prepared by following these steps:
- Mixing mesalazine and microcrystalline cellulose
- Granulating this mixture by the addition of hydroxypropyl methyl cellulose to obtain granule cores
- Obtaining enteric-coated granules by the spray drying coating of the granule cores with hypromellose acetate succinate having a pH solubility greater than or equal to 6.5
- Sieving the enteric-coated granules through a 200µm to 600µm mesh sieve
- Mixing the sieved enteric-coated granules with sucralose, xanthan gum, citric acid anhydrous, colloidal silicon dioxide and flavoring agent
- Dosing and filling the granulate into sachets

These analytically selected ratios ensure the required effective doses for the treatment and enhance stability, disintegration and dissolution profiles of the granulate composition subjected to the invention.

## Claims

1. An oral pharmaceutical composition in the form of granulate which is free of polyvinylpyrrolidone, comprising mesalazine or a pharmaceutically acceptable salt thereof in an amount of between 20-50% by weight of the composition; wherein the granulate composition comprises granule cores comprising mesalazine and an enteric coating on them and wherein the enteric coating is comprised of an inner layer containing hypromellose acetate succinate and an outer layer containing hypromellose acetate succinate.

2. The oral pharmaceutical composition according to claim 1, wherein the composition is also free of acrylic acid and methacrylic acid.

3. The oral pharmaceutical composition according to claim 1, wherein further suitable inner layer ingredients are selected from the group comprising hypromellose, lactose monohydrate, hydroxypropyl cellulose, polyvinyl alcohol, ethyl cellulose, polyethylene glycol, talc, triethyl citrate, polyvinyl alcohol-polyethylene glycol copolymers, ethyl cellulose dispersions, pigments, dyes, titanium dioxide, iron oxide and mixtures thereof.

4. The oral pharmaceutical composition according to claim 3, wherein the inner layer comprises hypromellose acetate succinate having a pH solubility greater than or equal to 6.5.

5. The oral pharmaceutical composition according to claim 1, wherein further suitable outer layer ingredients are selected from the group comprising hypromellose, lactose monohydrate, hydroxypropyl cellulose, polyvinyl alcohol, ethyl cellulose, polyethylene glycol, talc, triethyl citrate, polyvinyl alcohol-polyethylene glycol copolymers, ethyl cellulose dispersions, pigments, dyes, titanium dioxide, iron oxide and mixtures thereof.

6. The oral pharmaceutical composition according to claim 5, wherein the outer layer comprises hypromellose acetate succinate having a pH solubility greater than or equal to 6.0.

7. The oral pharmaceutical composition according to any preceding claims, the granulate composition further comprises at least one excipient selected from sweetening agent, suspending agent, buffer agent, flavoring agent, glidant, diluent, binder, coloring agent or mixtures thereof.

8. The oral pharmaceutical composition according to claim 7, wherein the granulate composition comprises;
- 20-50% by weight of mesalazine,
- 10-90% by weight of hypromellose acetate succinate having a pH solubility greater than or equal to 6.5,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent,
- 0.1-5% by weight of titanium dioxide

9. The oral pharmaceutical composition according to claim 7, wherein the granulate composition comprises;
- 20-50% by weight of mesalazine,
- 5-60% by weight of hypromellose acetate succinate having a pH solubility greater than or equal to 6.5,
- 5-30% by weight of hypromellose acetate succinate having a pH solubility greater than or equal to 6.0,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent,
- 0.1-5% by weight of titanium dioxide

10. The oral pharmaceutical composition according to claim 7, wherein the granulate composition comprises;
- 20-50% by weight of mesalazine,
- 1-30% by weight of microcrystalline cellulose,
- 1-30% by weight of hydroxypropyl methyl cellulose,
- 10-90% by weight of hypromellose acetate succinate having a pH solubility greater than or equal to 6.5,
- 1-20% by weight of sucralose,
- 0.01-5% by weight of xanthan gum,
- 0.01-5% by weight of citric acid anhydrous,
- 0.01-5% by weight of colloidal silicon dioxide,
- 0.1-10% by weight of flavoring agent

## Patentansprüche

1. Eine orale pharmazeutische Zusammensetzung in Form eines Granulats, das frei von Polyvinylpyrrolidon ist, umfassend Mesalazin oder ein pharmazeutisch verträgliches Salz davon in einer Menge von 20 bis 50 Gew.-% der Zusammensetzung; wobei die Granulatzusammensetzung Granulatkernpartikel, die Mesalazin enthalten, und eine darauf befindliche magensaftresistente Beschichtung umfasst und wobei die magensaftresistente Beschichtung aus einer inneren Schicht, die Hypromelloseacetatsuccinat enthält, und einer äußeren Schicht, die Hypromelloseacetatsuccinat enthält, besteht.

2. Orale pharmazeutische Zusammensetzung nach Anspruch 1, wobei die Zusammensetzung zudem frei von Acrylsäure und Methacrylsäure ist.

3. Die orale pharmazeutische Zusammensetzung nach Anspruch 1, wobei weitere geeignete Bestandteile der inneren Schicht aus der Gruppe ausgewählt sind, die Hypromellose, Lactose-Monohydrat, Hydroxypropylcellulose, Polyvinylalkohol, Ethylcellulose, Polyethylenglykol, Talk, Triethylcitrat, Polyvinylalkohol-Polyethylenglykol-Copolymere, Ethylcellulose-Dispersionen, Pigmente, Farbstoffe, Titandioxid, Eisenoxid und Mischungen davon umfasst.

4. Orale pharmazeutische Zusammensetzung nach Anspruch 3, wobei die innere Schicht Hypromelloseacetatsuccinat mit einer pH-Löslichkeit von größer oder gleich 6,5 umfasst.

5. Die orale pharmazeutische Zusammensetzung nach Anspruch 1, wobei weitere geeignete Bestandteile der äußeren Schicht aus der Gruppe ausgewählt sind, die aus Hypromellose, Lactose-Monohydrat, Hydroxypropylcellulose, Polyvinylalkohol, Ethylcellulose, Polyethylenglykol, Talkum, Triethylcitrat, Polyvinylalkohol-Polyethylenglykol-Copolymeren, Ethylcellulose-Dispersionen, Pigmenten, Farbstoffen, Titandioxid, Eisenoxid und Mischungen davon besteht.

6. Orale pharmazeutische Zusammensetzung nach Anspruch 5, wobei die äußere Schicht Hypromelloseacetatsuccinat mit einer pH-Löslichkeit von größer oder gleich 6,0 umfasst.

7. Orale pharmazeutische Zusammensetzung nach einem der vorstehenden Ansprüche, wobei die Granulatzusammensetzung ferner mindestens einen Hilfsstoff umfasst, der aus Süßungsmitteln, Suspensionsmitteln, Puffermitteln, Aromastoffen, Fließregulierungsmittel, Verdünnungsmitteln, Bindemitteln, Farbstoffen oder Mischungen davon ausgewählt ist.

8. Orale pharmazeutische Zusammensetzung nach Anspruch 7, wobei die Granulatzusammensetzung umfasst:
- 20-50 Gew.-% Mesalazin,
- 10-90 Gew.-% Hypromelloseacetatsuccinat mit einer pH-Löslichkeit von größer oder gleich 6,5,
- 1-20 Gew.-% Sucralose,
- 0,01-5 Gew.-% Xanthangummi,
- 0,01-5 Gew.-% wasserfreies Zitronensäure,
- 0,01-5 Gew.-% kolloidales Siliciumdioxid,
- 0,1-10 Gew.-% Aromastoff,
- 0,1-5 Gew.-% Titandioxid

9. Orale pharmazeutische Zusammensetzung nach Anspruch 7, wobei die Granulatzusammensetzung umfasst:
- 20-50 Gew.-% Mesalazin,
- 5-60 Gew.-% Hypromelloseacetatsuccinat mit einer pH-Löslichkeit von größer oder gleich 6,5,
- 5-30 Gew.-% Hypromelloseacetatsuccinat mit einer pH-Löslichkeit von größer oder gleich 6,0,
- 1-20 Gew.-% Sucralose,
- 0,01-5 Gew.-% Xanthangummi,
- 0,01-5 Gew.-% wasserfreie Zitronensäure,
- 0,01-5 Gew.-% kolloidales Siliciumdioxid,
- 0,1-10 Gew.-% Aromastoff,
- 0,1-5 Gew.-% Titandioxid

10. Orale pharmazeutische Zusammensetzung nach Anspruch 7, wobei die Granulatzusammensetzung umfasst:
- 20-50 Gew.-% Mesalazin,
- 1-30 Gew.-% mikrokristalline Cellulose,
- 1-30 Gew.-% Hydroxypropylmethylcellulose,
- 10-90 Gew.-% Hypromelloseacetatsuccinat mit einer pH-Löslichkeit von größer oder gleich 6,5,
- 1-20 Gew.-% Sucralose,
- 0,01-5 Gew.-% Xanthangummi,
- 0,01-5 Gew.-% wasserfreie Zitronensäure,
- 0,01-5 Gew.-% kolloidales Siliciumdioxid,
- 0,1-10 Gew.-% Aromastoff

## Revendications

1. Composition pharmaceutique orale sous forme de granulés qui est exempte de polyvinylpyrrolidone, comprenant de la mésalazine ou un sel pharmaceutiquement acceptable de celle-ci en une quantité comprise entre 20 et 50 % en poids de la composition ; dans laquelle la composition de granulés comprend des noyaux de granulés comprenant de la mésalazine et un enrobage entérique sur ceux-ci, et dans laquelle l'enrobage entérique est composé d'une couche interne contenant du succinate d'acétate d'hypromellose et d'une couche externe contenant du succinate d'acétate d'hypromellose.

2. Composition pharmaceutique orale selon la revendication 1, dans laquelle la composition est également exempte d'acide acrylique et d'acide méthacrylique.

3. Composition pharmaceutique orale selon la revendication 1, dans laquelle d'autres ingrédients de couche interne appropriés sont choisis dans le groupe comprenant l'hypromellose, le lactose monohydraté, l'hydroxypropylcellulose, l'alcool polyvinylique, l'éthylcellulose, le polyéthylène glycol, le talc, le citrate de triéthyle, les copolymères d'alcool polyvinylique-polyéthylène glycol, les dispersions d'éthylcellulose, les pigments, les colorants, le dioxyde de titane, l'oxyde de fer et leurs mélanges.

4. Composition pharmaceutique orale selon la revendication 3, dans laquelle la couche interne comprend du succinate d'acétate d'hypromellose ayant une solubilité au pH supérieure ou égale à 6,5.

5. Composition pharmaceutique orale selon la revendication 1, dans laquelle d'autres ingrédients de couche externe appropriés sont choisis dans le groupe comprenant l'hypromellose, le lactose monohydraté, l'hydroxypropylcellulose, l'alcool polyvinylique, l'éthylcellulose, le polyéthylène glycol, le talc, le citrate de triéthyle, les copolymères d'alcool polyvinylique-polyéthylène glycol, les dispersions d'éthylcellulose, les pigments, les colorants, le dioxyde de titane, l'oxyde de fer et leurs mélanges.

6. Composition pharmaceutique orale selon la revendication 5, dans laquelle la couche externe comprend du succinate d'acétate d'hypromellose ayant une solubilité au pH supérieure ou égale à 6,0.

7. Composition pharmaceutique orale selon l'une quelconque des revendications précédentes, dans laquelle la composition de granulés comprend en outre au moins un excipient choisi parmi un édulcorant, un agent de suspension, un agent tampon, un agent aromatisant, un glissant, un diluant, un liant, un agent de coloration ou leurs mélanges.

8. Composition pharmaceutique orale selon la revendication 7, dans laquelle la composition de granulés comprend ;
- 20-50 % en poids de mésalazine,
- 10-90 % en poids de succinate d'acétate d'hypromellose ayant une solubilité au pH supérieure ou égale à 6,5,
- 1-20 % en poids de sucralose,
- 0,01-5 % en poids de gomme xanthane,
- 0,01-5 % en poids d'acide citrique anhydre,
- 0,01-5 % en poids de dioxyde de silicium colloïdal,
- 0,1-10 % en poids d'agent aromatisant,
- 0,1-5 % en poids de dioxyde de titane.

9. Composition pharmaceutique orale selon la revendication 7, dans laquelle la composition de granulés comprend ;
- 20-50 % en poids de mésalazine,
- 5-60 % en poids de succinate d'acétate d'hypromellose ayant une solubilité au pH supérieure ou égale à 6,5,
- 5-30 % en poids de succinate d'acétate d'hypromellose ayant une solubilité au pH supérieure ou égale à 6,0,
- 1-20 % en poids de sucralose,
- 0,01-5 % en poids de gomme xanthane,
- 0,01-5 % en poids d'acide citrique anhydre,
- 0,01-5 % en poids de dioxyde de silicium colloïdal,
- 0,1-10 % en poids d'agent aromatisant,
- 0,1-5 % en poids de dioxyde de titane.

10. Composition pharmaceutique orale selon la revendication 7, dans laquelle la composition de granulés comprend ;
- 20-50 % en poids de mésalazine,
- 1-30 % en poids de cellulose microcristalline,
- 1-30 % en poids d'hydroxypropylméthylcellulose,
- 10-90 % en poids de succinate d'acétate d'hypromellose ayant une solubilité au pH supérieure ou égale à 6,5,
- 1-20 % en poids de sucralose,
- 0,01-5 % en poids de gomme xanthane,
- 0,01-5 % en poids d'acide citrique anhydre,
- 0,01-5 % en poids de dioxyde de silicium colloïdal,
- 0,1-10 % en poids d'agent aromatisant.
